# EUROPEAN PATENT APPLICATION

(11) **EP 1 130 030 A1**
(43) Date of publication of application: **05.09.2001**
(21) Application number: 01104821.2
(22) Date of filing: 27.02.2001
(51) Int. Cl.: C07K 14/575, C12N 15/00

(54) **Human erythroid differentiation related factor**

(30) Priority: 02.03.2000 US 517225
(71) Applicant: Roche Diagnostics Corporation, Indianapolis, IN 46250 (US)
(72) Inventor: Xu, Hongxia, Castro Valley, CA 94552 (US); Mahoney, Walter, Benicia, CA 94510 (US); Schueler, Paula, Benicia, CA 94510 (US); Harriman, William D., Alameda, CA 94501 (US)
(74) Representative: Schwarz, Ralf

(57) **Abstract**

The invention provides a new protein, human erythroid differentiation related factor (hEDRF), polynucleotides encoding the protein, and antibodies which specifically bind the protein. Also provided are methods for the treatment anemias and other erythrocyte deficiencies.

SEQUENCE LISTING

<110> Xu, Hongxia
      Schueler, Paula
      Harriman, Bill
      Mahoney, Walt

<120> Human Erythrocyte Differentiation
  Related Factor

<130> 337462003200

<140> Unassigned
<141> 2000-03-02

<160> 9

<170> FastSEQ for Windows Version 3.0

<210> 1
<211> 597
<212> DNA
<213> Homo sapien

<400> 1
ggcacgagct gacacttgac tccttgccta catctatagc ttggcacaga gagattcacg      60
caccctcaag agtgtgggtg agacatatac agcctgttag acctgaaggc agatggctct     120
tcttaaggcc aataaggatc tcatttccgc aggattgaag gagttcagcg ttctgctgaa     180
tcagcaggtc ttcaatgatc ctctcgtctc tgaagaagac atggtgactg tggtggagga     240
ctggatgaac ttctacatca actattacag gcagcaggtg acaggggagc cccaagagcg     300
agacaaggct ctgcaggagc ttcggcaaga gctgaacact ctggccaacc ctttcctggc     360
caagtacagg gacttcctga agtctcatga gctcccgagt cacccaccgc cctcctccta     420
gctcagggac ccagcccctc ctctctgaga aactctgacc ttcatgtcct taggctgtgc     480
tcctgccact ctaccctgac acctcaataa agaccagtgc tggttttgtt ggactaaaaa     540
aaaaaaaaaa aaaaaaaaaa aaaaaaaaaa aaaaaaaaaa aaaaaaaaaa aaaaaaa        597

<210> 2
<211> 102
<212> PRT
<213> Homo sapien

<400> 2
Met Ala Leu Leu Lys Ala Asn Lys Asp Leu Ile Ser Ala Gly Leu Lys
 1               5                  10                  15
Glu Phe Ser Val Leu Leu Asn Gln Gln Val Phe Asn Asp Pro Leu Val
            20                  25                  30
Ser Glu Glu Asp Met Val Thr Val Val Glu Asp Trp Met Asn Phe Tyr
        35                  40                  45
Ile Asn Tyr Tyr Arg Gln Gln Val Thr Gly Glu Pro Gln Glu Arg Asp
    50                  55                  60
Lys Ala Leu Gln Glu Leu Arg Gln Glu Leu Asn Thr Leu Ala Asn Pro
65                  70                  75                  80
Phe Leu Ala Lys Tyr Arg Asp Phe Leu Lys Ser His Glu Leu Pro Ser
                85                  90                  95
His Pro Pro Pro Ser Ser
            100

<210> 3
<211> 505
<212> DNA
<213> Mus musculus

<220>
<221> CDS
<222> (53)...(361)

<400> 3
tcaaaccccg aggattgtgg atgaggcggg ctcagcacca ttagacttga ag atg gcc 58
Met Ala
1

cct ttt cag agc aat aag gat ctg atc tcc aca ggg ata aag gag ttt 106
Pro Phe Gln Ser Asn Lys Asp Leu Ile Ser Thr Gly Ile Lys Glu Phe
        5                   10                  15

aat gtt ctg ctg gat cag cag gtc ttt gat gat cct ctg atc tct gaa 154
Asn Val Leu Leu Asp Gln Gln Val Phe Asp Asp Pro Leu Ile Ser Glu
    20                  25                  30

gaa gac atg gtg att gtg gtt cat gac tgg gtg aac ttg tac acc aac 202
Glu Asp Met Val Ile Val Val His Asp Trp Val Asn Leu Tyr Thr Asn
35                  40                  45                  50

tat tac aag aag ctc gtg cat ggg gag cag gag gag caa gac agg gcc 250
Tyr Tyr Lys Lys Leu Val His Gly Glu Gln Glu Glu Gln Asp Arg Ala
                55                  60                  65

atg aca gaa ttc cag caa gag ctg agt act ctg ggc agc cag ttt cta 298
Met Thr Glu Phe Gln Gln Glu Leu Ser Thr Leu Gly Ser Gln Phe Leu
            70                  75                  80

gcc aaa tac agg acc ttt ctg aag tcc aaa gag ccc cca agc aat aca 346
Ala Lys Tyr Arg Thr Phe Leu Lys Ser Lys Glu Pro Pro Ser Asn Thr
        85                  90                  95

ctg ccc tcc tca taa cttaaagggt ctgggcatca tgtcttagaa ccccaaacac 401
Leu Pro Ser Ser *
    100

tcggctctgt gttatatctt cagaccgttc tcccaagatg ttgctgtact ttgacatgcc 461
aataaagacc aaatactcaa aaaaaaaaaa aaaaaaaaaa aaaa 505

<210> 4
<211> 102
<212> PRT
<213> Mus musculus

<400> 4
Met Ala Pro Phe Gln Ser Asn Lys Asp Leu Ile Ser Thr Gly Ile Lys
1               5                   10                  15
Glu Phe Asn Val Leu Leu Asp Gln Gln Val Phe Asp Asp Pro Leu Ile
            20                  25                  30
Ser Glu Glu Asp Met Val Ile Val Val His Asp Trp Val Asn Leu Tyr
        35                  40                  45
Thr Asn Tyr Tyr Lys Lys Leu Val His Gly Glu Gln Glu Glu Gln Asp
    50                  55                  60
Arg Ala Met Thr Glu Phe Gln Gln Glu Leu Ser Thr Leu Gly Ser Gln
65                  70                  75                  80
Phe Leu Ala Lys Tyr Arg Thr Phe Leu Lys Ser Lys Glu Pro Pro Ser
                85                  90                  95
Asn Thr Leu Pro Ser Ser
            100

<210> 5
<211> 744

<212> DNA
<213> Homo sapien

<220>
<223> Anti-hEDRF anitbody e9

<221> CDS
<222> (1)...(744)

<400> 5
gag gtg cag ctg gtg gag tct ggg gga ggc ttg gta cag cct ggc agg 48
Glu Val Gln Leu Val Glu Ser Gly Gly Gly Leu Val Gln Pro Gly Arg
1               5                   10                  15

tcc ctg aga ctc tcc tgt gca gcc tct gga ttc acc ttt gat gat tat 96
Ser Leu Arg Leu Ser Cys Ala Ala Ser Gly Phe Thr Phe Asp Asp Tyr
            20                  25                  30

gcc atg cac tgg gtc cgg caa gct cca ggg aag ggc ctg gag tgg gtc 144
Ala Met His Trp Val Arg Gln Ala Pro Gly Lys Gly Leu Glu Trp Val
        35                  40                  45

tca ggt att agt tgg aat agt ggt agc ata ggc tat gcg gac tct gtg 192
Ser Gly Ile Ser Trp Asn Ser Gly Ser Ile Gly Tyr Ala Asp Ser Val
    50                  55                  60

aag ggc cga ttc acc atc tcc aga gac aac gcc aag aac tcc ctg tat 240
Lys Gly Arg Phe Thr Ile Ser Arg Asp Asn Ala Lys Asn Ser Leu Tyr
65                  70                  75                  80

ctg caa atg aac aga ctg aga gct gag gac acg gcc gtg tat tac tgt 288
Leu Gln Met Asn Arg Leu Arg Ala Glu Asp Thr Ala Val Tyr Tyr Cys
                85                  90                  95

gca aga gag agt tct gat ttg gag tct aat gtg act tgg ggc caa ggt 336
Ala Arg Glu Ser Ser Asp Leu Glu Ser Asn Val Thr Trp Gly Gln Gly
            100                 105                 110

acc ctg gtc acc gtc tcg agt ggt gga ggc ggt tca ggc gga ggt ggc 384
Thr Leu Val Thr Val Ser Ser Gly Gly Gly Gly Ser Gly Gly Gly Gly
        115                 120                 125

tct ggc ggt agt gca ctt gat gtt gtg atg act cag tct cca ctc tcc 432
Ser Gly Gly Ser Ala Leu Asp Val Val Met Thr Gln Ser Pro Leu Ser
    130                 135                 140

ctg ccc gtc acc cct gga gag ccg gcc tcc atc tcc tgc agg tct agt 480
Leu Pro Val Thr Pro Gly Glu Pro Ala Ser Ile Ser Cys Arg Ser Ser
145                 150                 155                 160

cag agc ctc ctg cat agt aat gga tac aac tat ttg gat tgg tac ctg 528
Gln Ser Leu Leu His Ser Asn Gly Tyr Asn Tyr Leu Asp Trp Tyr Leu
                165                 170                 175

cag aag cca ggg cag tct cca cag ctc ctg atc tat ttg ggt tct aat 576
Gln Lys Pro Gly Gln Ser Pro Gln Leu Leu Ile Tyr Leu Gly Ser Asn
            180                 185                 190

cgg gcc tcc ggg gtc cct gac agg ttc agt ggc agt gga tca ggc aca 624
Arg Ala Ser Gly Val Pro Asp Arg Phe Ser Gly Ser Gly Ser Gly Thr
        195                 200                 205

gat ttt aca ctg aaa atc agc aga gtg gag gct gag gat gtt ggg gtt 672
Asp Phe Thr Leu Lys Ile Ser Arg Val Glu Ala Glu Asp Val Gly Val
    210                 215                 220

tat tac tgc atg caa gct cta caa act ccc tct ctt acg ttc ggc caa 720
Tyr Tyr Cys Met Gln Ala Leu Gln Thr Pro Ser Leu Thr Phe Gly Gln
225                 230                 235                 240

ggg acc aag ctg gaa atc aaa cgt 744
Gly Thr Lys Leu Glu Ile Lys Arg
                245

<210> 6
<211> 248
<212> PRT
<213> Homo sapien

<400> 6
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Gly Ile Ser Trp Asn Ser Gly Ser Ile Gly Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Arg Leu Arg Ala Glu Asp Thr Ala Val Tyr Tyr Cys
                85                  90                  95
Ala Arg Glu Ser Ser Asp Leu Glu Ser Asn Val Thr Trp Gly Gln Gly
            100                 105                 110
Thr Leu Val Thr Val Ser Ser Gly Gly Gly Gly Ser Gly Gly Gly Gly
        115                 120                 125
Ser Gly Gly Ser Ala Leu Asp Val Val Met Thr Gln Ser Pro Leu Ser
    130                 135                 140
Leu Pro Val Thr Pro Gly Glu Pro Ala Ser Ile Ser Cys Arg Ser Ser
145                 150                 155                 160
Gln Ser Leu Leu His Ser Asn Gly Tyr Asn Tyr Leu Asp Trp Tyr Leu
                165                 170                 175
Gln Lys Pro Gly Gln Ser Pro Gln Leu Leu Ile Tyr Leu Gly Ser Asn
            180                 185                 190
Arg Ala Ser Gly Val Pro Asp Arg Phe Ser Gly Ser Gly Ser Gly Thr
        195                 200                 205
Asp Phe Thr Leu Lys Ile Ser Arg Val Glu Ala Glu Asp Val Gly Val
    210                 215                 220
Tyr Tyr Cys Met Gln Ala Leu Gln Thr Pro Ser Leu Thr Phe Gly Gln
225                 230                 235                 240
Gly Thr Lys Leu Glu Ile Lys Arg
                245

<210> 7
<211> 729
<212> DNA
<213> Homo sapien

<220>
<223> Anti-hEDRF antibody e11

<221> CDS
<222> (1)...(729)

<221> variation
<222> (622)...(622)
<223> N= any nucleotide

<221> variation
<222> (646)...(646)
<223> N= any nucleotide

<221> variation
<222> (662)...(663)
<223> N= any nucleotide

<221> variation
<222> (688)...(688)
<223> N= any nucleotide

<400> 7
tcc cag gtg cag ctg gtg cag tct ggg gga ggc ctg gta cag cct ggg 48
Ser Gln Val Gln Leu Val Gln Ser Gly Gly Gly Leu Val Gln Pro Gly
1               5                   10                  15

ggg tcc ctg aga ctc tcc tgt gca gcc tct gga ttc acc ttt gat gat 96
Gly Ser Leu Arg Leu Ser Cys Ala Ala Ser Gly Phe Thr Phe Asp Asp
            20                  25                  30

tat gcc atg cac tgg gtc cgg caa gct cca ggg aag ggc ctg gag tgg 144
Tyr Ala Met His Trp Val Arg Gln Ala Pro Gly Lys Gly Leu Glu Trp
        35                  40                  45

gtc tca ggt att agt tgg aat agt ggt agc ata ggc tat gcg gac tct 192
Val Ser Gly Ile Ser Trp Asn Ser Gly Ser Ile Gly Tyr Ala Asp Ser
    50                  55                  60

gtg aag ggc cga ttc acc atc tcc aga gac aac gcc aag aac aca ctg 240
Val Lys Gly Arg Phe Thr Ile Ser Arg Asp Asn Ala Lys Asn Thr Leu
65                  70                  75                  80

tat ctg caa atg aca ctg aga gtc gag gac acg gct gtg tat tac tgt 288
Tyr Leu Gln Met Thr Leu Arg Val Glu Asp Thr Ala Val Tyr Tyr Cys
                85                  90                  95

tca aga gat ctg ggg ggg gca gat gac tcc tgg ggc cag gga acc ctg 336
Ser Arg Asp Leu Gly Gly Ala Asp Asp Ser Trp Gly Gln Gly Thr Leu
            100                 105                 110

gtc acc gtc tcc tca ggt gga ggc ggt tca ggc gga ggt ggc tct ggc 384
Val Thr Val Ser Ser Gly Gly Gly Gly Ser Gly Gly Gly Gly Ser Gly
        115                 120                 125

ggt ggc gga tcg cag tct gct ctg act cag cct cgc tca gtg tcc ggg 432
Gly Gly Gly Ser Gln Ser Ala Leu Thr Gln Pro Arg Ser Val Ser Gly
    130                 135                 140

tct cct gga cag tca gtc acc atc tcc tgc act gga acc agc agt gat 480
Ser Pro Gly Gln Ser Val Thr Ile Ser Cys Thr Gly Thr Ser Ser Asp
145                 150                 155                 160

gtt ggt ggt tat aac tat gtc tcc tgg tat caa cag cac cca ggc aaa 528
Val Gly Gly Tyr Asn Tyr Val Ser Trp Tyr Gln Gln His Pro Gly Lys
                165                 170                 175

gcc ccc aaa ctc atg att tat gag gtc agt aat cgg ccc cca ggg gtc 576
Ala Pro Lys Leu Met Ile Tyr Glu Val Ser Asn Arg Pro Pro Gly Val

180                 185                 190

cct gat cgc ttc tct ggg tcc aag tct ggc aac acg gcc tcc ctg ncc       624
Pro Asp Arg Phe Ser Gly Ser Lys Ser Gly Asn Thr Ala Ser Leu Xaa
        195                 200                 205

atc tcg ggg ctc cag act gag nac gag ggt gat tat tnn tgc agc tca       672
Ile Ser Gly Leu Gln Thr Glu Xaa Glu Gly Asp Tyr Xaa Cys Ser Ser
    210                 215                 220

tat aca agc agc agc nct tat gtc ttc gga act ggg acc aag ctg acc       720
Tyr Thr Ser Ser Ser Xaa Tyr Val Phe Gly Thr Gly Thr Lys Leu Thr
225                 230                 235                 240

gtc cta ggt                                                           729
Val Leu Gly

<210> 8
<211> 243
<212> PRT
<213> Homo sapien

<400> 8
Ser Gln Val Gln Leu Val Gln Ser Gly Gly Gly Leu Val Gln Pro Gly
 1               5                  10                  15
Gly Ser Leu Arg Leu Ser Cys Ala Ala Ser Gly Phe Thr Phe Asp Asp
            20                  25                  30
Tyr Ala Met His Trp Val Arg Gln Ala Pro Gly Lys Gly Leu Glu Trp
        35                  40                  45
Val Ser Gly Ile Ser Trp Asn Ser Gly Ser Ile Gly Tyr Ala Asp Ser
    50                  55                  60
Val Lys Gly Arg Phe Thr Ile Ser Arg Asp Asn Ala Lys Asn Thr Leu
65                  70                  75                  80
Tyr Leu Gln Met Thr Leu Arg Val Glu Asp Thr Ala Val Tyr Tyr Cys
                85                  90                  95
Ser Arg Asp Leu Gly Gly Ala Asp Asp Ser Trp Gly Gln Gly Thr Leu
            100                 105                 110
Val Thr Val Ser Ser Gly Gly Gly Gly Ser Gly Gly Gly Gly Ser Gly
        115                 120                 125
Gly Gly Gly Ser Gln Ser Ala Leu Thr Gln Pro Arg Ser Val Ser Gly
    130                 135                 140
Ser Pro Gly Gln Ser Val Thr Ile Ser Cys Thr Gly Thr Ser Ser Asp
145                 150                 155                 160
Val Gly Gly Tyr Asn Tyr Val Ser Trp Tyr Gln Gln His Pro Gly Lys
                165                 170                 175
Ala Pro Lys Leu Met Ile Tyr Glu Val Ser Asn Arg Pro Pro Gly Val
            180                 185                 190
Pro Asp Arg Phe Ser Gly Ser Lys Ser Gly Asn Thr Ala Ser Leu Xaa
        195                 200                 205
Ile Ser Gly Leu Gln Thr Glu Xaa Glu Gly Asp Tyr Xaa Cys Ser Ser
    210                 215                 220
Tyr Thr Ser Ser Ser Xaa Tyr Val Phe Gly Thr Gly Thr Lys Leu Thr
225                 230                 235                 240
Val Leu Gly

<210> 9
<211> 48
<212> PRT
<213> Homo sapien

<220>
<223> Purified 345-7a peptide

<400> 9
Asn Tyr Tyr Arg Gln Gln Val Thr Gly Glu Pro Gln Glu Arg Asp Lys
1               5                   10                  15
Ala Leu Gln Glu Leu Arg Gln Glu Leu Asn Thr Leu Ala Asn Pro Phe
            20                  25                  30
Leu Ala Lys Tyr Arg Asp Phe Leu Lys Ser His Glu Leu Pro Ser His
        35                  40                  45

1

1

## Description

### TECHNICAL FIELD

The invention relates to polynucleotides and polypeptides occurring in erythrocytes or their progenitors, and more particularly to a new protein, human erythroid differentiation related factor (hEDRF), polynucleotides encoding hEDRF, antibodies specific for hEDRF, and their uses.

### BACKGROUND ART

The differentiation of cells along the erythroid pathway to mature erythrocytes is essential to human homeostasis. A number of human disease conditions relate to abnormal depletion of erythrocytes, and could benefit from promotion of erythrocyte development. Other human disease conditions relate to abnormal hyperplasia of blood cells or their precursors. It is beneficial to have new reagents to locate or correct such abnormal events.

Erythroid cells are generated from a small number of pluripotent hematopoietic stem cells which are established at a series of different sites during vertebrate development. Fetal erythroid cell production is first located in the blood islands of the yolk sack. The cells produced here, termed "primitive" erythroid cells, are nucleated and are present only transiently during development. The liver takes over as the primary site of erythroid cell production as development proceeds. The liver-produced erythroid cells are termed "definitive" erythroid cells which, in mammals, enucleate to produce fetal erythrocytes. Shortly before birth, the primary site of erythrogenesis moves to bone marrow, its permanent site.

Definitive erythrocytes are the final product of one pathway of differentiation from pluripotent stem cells, which are the source of all blood cells. Pluripotent stem cells give rise to CFU-S (colony forming unit-spleen), which give rise to CFU-GEMM (colony forming unit-granulocyte, erythrocyte, monocyte and megakaryocyte). BFU-E are derived from CFU-GEMM, and are committed to the erythrocyte differentiation pathway. Next in the developmental pathway are CFU-E, followed by proerythroblasts, which give rise to a series of types of erythroblast, differentiated on the basis of histologic staining. Erythroblasts give rise to reticulocytes, which enucleate to become erythrocytes.

### SUMMARY OF THE INVENTION

A new factor has been found that is associated with cells in the erythroid differentiation pathway. Messenger RNA has been obtained from erythrocyte precursors which contains an open reading frame (ORF) of about 102 amino acids. The protein encoded by this ORF has been given the name human erythroid differentiation related factor (hEDRF). The invention provides hEDRF and hEDRF-related polypeptides, polynucleotides encoding such polypeptides, as well as reagents which can be used to detect hEDRF protein and mRNA and the location and identity of cells producing it.

In one embodiment, the invention provides hEDRF polypeptides. The hEDRF polypeptides provided by the invention include pro-hEDRDF *(i.e.,* amino acids 1-102 of SEQ. ID. NO:2) and processed forms thereof (*e.g.*, amino acids 6-102, 12-102, 13-102, 14-102, and 15-102 of SEQ. ID. NO:2), as well as polypeptides related to hEDRF. The polypeptides of this embodiment may comprise a segment of at least about 5 consecutive amino acids that are identical to consecutive amino acids shown in the hEDRF sequence, but not the mouse homologue (mEDRF). This embodiment further includes polypeptides which have at least about 65% identity to and/or 85% similarity to pro-hEDRF and/or processed forms thereof.

In another embodiment, the invention provides polynucleotides which encode hEDRF as well as hEDRF-related polypeptides. Exemplary are polynucleotides comprising at least about 25 consecutive nucleotides that are identical to a sequence in the hEDRF cDNA, and/or that encode one of the polypeptides of this invention. Also included within the invention are isolated polynucleotides which hybridize with DNA or RNA encoding hEDRF, including the sequence of SEQ ID NO:1, particularly nucleotides 113-418 of SEQ ID NO:1 and complements thereof. The polynucleotides of the invention may be used to detect hEDRF expression at the mRNA level (*e.g.*, in human erythroid cells) or to produce hEDRF or hEDRF-related polypeptides.

Further embodiments include recombinant host cells which comprise a polynucleotide of the invention, and recombinant host cells which comprise an expression construct which comprises a polynucleotide of the invention.

Another embodiment of the invention provides antibodies specific for hEDRF and the other polypeptides of this invention. The antibodies may be used, for example, to separate or detect the corresponding antigen (*e.g.,* for detection/quantitation of hEDRF in samples taken from human subjects).

### BRIEF DESCRIPTION OF THE DRAWINGS

FIG. 1 is a map of cloned cDNA obtained from human tissue, with an encoding region for hEDRF.

FIG. 2 is a representation of the protein sequence for hEDRF (SEQ. ID NO:2), along with some features of potential interest predicted by computer algorithm. Upper panel shows the potential signal peptidase cleavage sites; lower panel shows potential functional motifs.

FIG. 3 is a comparison of the protein sequence for hEDRF (SEQ. ID NO:2) compared with the mouse homologue (SEQ. ID NO:4). Residues identical between the homologues are represented by a period (.). The sequences are about 63% identical.

FIG. 4 shows the nucleotide sequence and deduced amino acid sequence of anti-hEDRF antibody e9 (SEQ ID NOS: 5-6). Position numbers refer to nucleotide sequence.

FIG. 5 shows the nucleotide sequence and deduced amino acid sequence of anti-hEDRF antibody e11 (SEQ ID NOS:7-8). Position numbers refer to nucleotide sequence.

FIG. 6 shows a reproduction of a Northern blot, wherein a selection of tissues were probed for hEDRF expression. From left to right, the lanes were loaded with an RNA molecular weight marker, a blank, a pool of adult liver RNA (AL 2/3), a pool of week 16 fetal liver RNA, a pool of adult bone marrow RNA (BM7), a pool of adult peripheral blood RNA (PBA3) prepared from a lysed sample, a pool of adult peripheral blood RNA (PBA1) prepared from a non-lysed sample, a pool of fetal blood RNA from 10 to 12 week fetal liver, and a pool of RNA from term placenta.

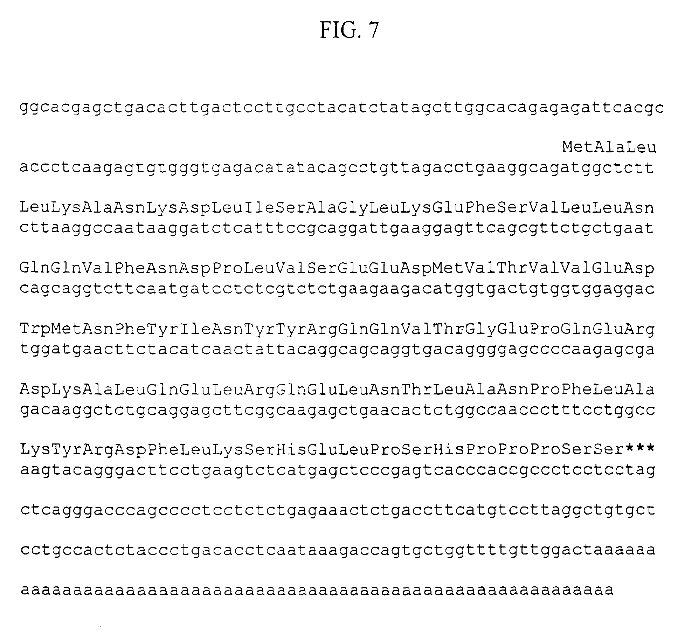

FIG. 7 shows the complete human cDNA for hEDRF, along with translation of the second open reading frame (ORF2), which encodes hEDRF protein. Three asterisks ("^{***}") indicates the termination codon.

### BEST MODE FOR CARRYING OUT THE INVENTION

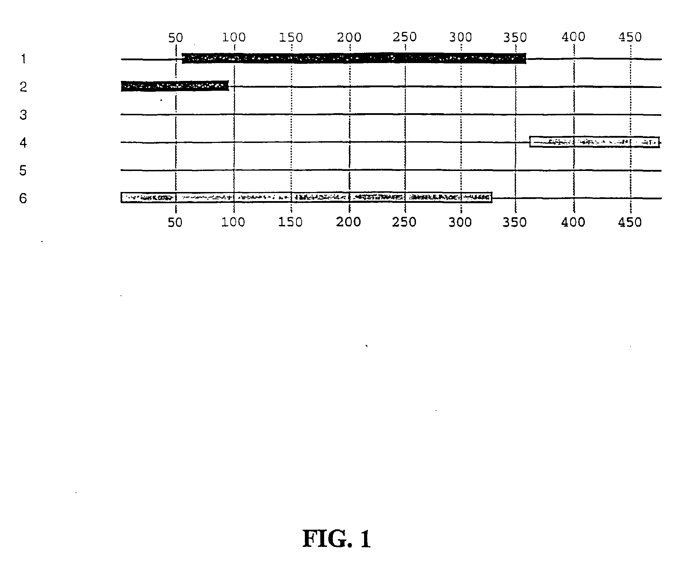

The inventors have isolated a novel cDNA from a cDNA library which was created using human tissue enriched for cells in the erythroid differentiation pathway (SEQ. ID NO:1). A map of some of the open reading frames (ORFs) is shown in FIG. 1: ORF 1-3 are in the forward direction, and ORF 4-6 are in the antisense direction. ORF 2 encodes a protein of 102 amino acids, and the protein encoded by that ORF has been designated human erythroid differentiation related factor, or hEDRF (SEQ. ID NO:2).

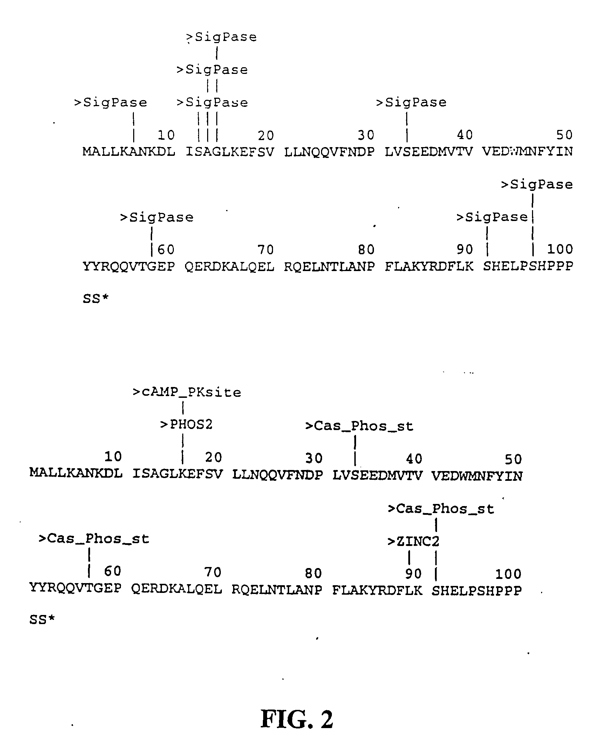

FIG. 2 is a representation of features in the translated hEDRF amino acid sequence, predicted by the computer algorithm MacVector™ (Version 6.0, obtained from Oxford Molecular). There are a number of potential signal peptidase cleavage sites near the N-terminus. There are also cAMP dependent PK phosphorylation and casein kinase sites along the sequence, and a zinc finger motif near the C-terminus.

This invention includes polynucleotides, polypeptides, and antibodies related to hEDRF and their use. Further description is provided in the sections that follow.

### Definitions

The terms "polynucleotide" and "oligonucleotide" are used interchangeably, and refer to a polymeric form of nucleotides of any length, either deoxyribonucleotides or ribonucleotides, or analogs. Included are genes and gene fragments, mRNA, tRNA, rRNA, ribozymes, cDNA, recombinant polynucleotides, branched polynucleotides, plasmids, vectors, isolated DNA and RNA, nucleic acid probes, and primers. A polynucleotide may comprise modified nucleotides, such as methylated nucleotides and nucleotide analogs; modifications may be imparted before or after assembly of the polymer. The sequence of nucleotides may also be interrupted by non-nucleotide components. The term polynucleotide, as used herein, refers interchangeably to double- and single-stranded molecules. Unless otherwise specified or required, any embodiment of the invention that is a polynucleotide encompasses both a double-stranded form, and each of two complementary single-stranded forms known or predicted to make up the double-stranded form.

"Hybridization" refers to a reaction in which one or more polynucleotides react to form a complex that is stabilized via hydrogen bonding between the bases of the nucleotide residues. The hydrogen bonding can occur by Watson-Crick base pairing, Hoogsteen binding, or in any other sequence-specific manner. A hybridization reaction may constitute a step in a more extensive process, such as the initiation of PCR amplification.

Hybridization reactions can be performed under conditions of different "stringency". Conditions that increase the stringency of a hybridization reaction are widely known (see e.g., Sambrook et al., *infra).* Examples of conditions in order of increasing stringency: incubation temperatures of 25°C, 37°C, 50°C, and 68°C; buffer concentrations of 10 x SSC, 6 x SSC, 1 x SSC, 0.1 x SSC (where SSC is 0.15 M NaCl and 15 mM citrate buffer) and their equivalent using other buffer systems; formamide concentrations of 0%, 25%, 50%, and 75%; incubation times from 5 min to 24 h; 1, 2, or more washing steps; wash incubation times of 1, 5, or 15 min; and wash solutions of 6 x SSC, 1 x SSC, 0.1 x SSC, or deionized water.

A "stable duplex" of polynucleotides, or a "stable complex" formed between any two or more components in a biochemical reaction, refers to a duplex or complex that is sufficiently long-lasting to persist between the formation of the duplex or complex, and its subsequent detection. Components of the duplex or complex may be reversibly or irreversibly associated, but the association must be able to withstand whatever conditions exist or are introduced between the moment of formation and the moment of detection.

A polynucleotide "probe" is a reagent for detecting a target polynucleotide potentially present in a sample of interest by a hybridization reaction. A "primer" is an oligonucleotide, generally with a free 3' -OH group, that hybridizes to a template polynucleotide in a sample of interest and thereafter promotes amplification of the template.

A "host cell" denotes a procaryotic or eucaryotic cell has been genetically altered, or is capable of being genetically altered by administration of an exogenous polynucleotide, such as a bacterial plasmid or recombinant vector. When referring to genetically altered cells, the term refers both to the originally altered cell and to the progeny thereof.

"Genetic alteration" refers to a process wherein a genetic element is introduced into a cell other than by mitosis or meiosis. Genetic alteration may be effected, for example, by transfecting a cell with a recombinant plasmid or other polynucleotide through any known process, such as electroporation, calcium phosphate precipitation, contacting with a polynucleotide-liposome complex, or by transduction or infection with a DNA or RNA virus or viral vector. A cell is said to be "inheritably altered" if a genetic alteration is introduced which is inheritable by progeny of the altered cell. Preferably, the genetic element is introduced into a chromosome or mini-chromosome in the cell.

The terms "polypeptide", "peptide" and "protein" are used interchangeably herein to refer to polymers of amino acids of any length. The polymer may comprise modified amino acids, it may be linear or branched, and it may be interrupted by non-amino acids. The terms also encompass an amino acid polymer that has been modified naturally or by intervention; for example, disulfide bond formation, glycosylation, lipidation, acetylation, phosphorylation.

A "fusion polypeptide" is a polypeptide comprising regions in a different position in the sequence than occurs in nature. The regions may normally exist in separate proteins and are brought together in the fusion polypeptide; or they may normally exist in the same protein but are placed in a new arrangement in the fusion polypeptide.

As used herein, the term "antibody" means an immunoglobulin molecule or a fragment of an immunoglobulin molecule having the ability to specifically bind to a particular antigen. Antibodies are well known to those of ordinary skill in the science of immunology. As used herein, the term "antibody" means not only intact antibody molecules but also fragments of antibody molecules retaining antigen binding ability. Such fragments are also well known in the art and are regularly employed both *in vitro* and *in vivo.* In particular, as used herein, the term "antibody" means not only intact immunoglobulin molecules of any isotype (IgA, IgG, IgE, IgD, IgM) but also the well-known active (i.e., antigen-binding) fragments F(ab')₂, Fab, Fv, scFv, Fd, V_{H} and V_{L}. For antibody fragments, see, for example "Immunochemistry in Practice" (Johnstone and Thorpe, eds.. 1996; Blackwell Science), p. 69. The term "antibody" further includes single chain antibodies, CDR-grafted antibodies, diabodies, chimeric antibodies, humanized antibodies, and a Fab expression library. The term also includes fusion polypeptides comprising an antibody of the invention and another polypeptide or a portion of a polypeptide (a "fusion partner"). Examples of fusion partners include biological response modifiers, lymphokines, cytokines, and cell surface antigens. "Antibody activity" refers to the ability of an antibody to bind a specific antigen in preference to other potential antigens via the antigen combining site located within a variable region of an immunoglobulin.

An "isolated" polynucleotide, polypeptide, protein, antibody, or other substance refers to a preparation of the substance in which some or all of the other components that are present with the substance in its natural form or present in the source from which the substance is obtained are removed or reduced. Thus, for example, an isolated polypeptide may be prepared by using a purification technique to enrich it from a source mixture. Enrichment can be measured on an absolute basis, such as weight per volume of solution, or it can be measured in relation to a second, potentially interfering substance present in the source mixture. Enrichments by 2, 10, 100, and 1000 fold are increasingly more preferred. A substance can also be provided in an isolated state by a process of artificial assembly, such as by chemical synthesis or recombinant expression.

A polynucleotide used in a reaction, such as a probe used in a hybridization reaction, a primer used in a PCR, or a polynucleotide present in a pharmaceutical preparation, is referred to as "specific" or "selective" if it hybridizes or reacts with the intended target more frequently, more rapidly, or with greater duration than it does with alternative substances. Similarly, an antibody is referred to as "specific" or "selective" if it binds via at least one antigen recognition site to the intended target more frequently, more rapidly, or with greater duration than it does to alternative substances.

It is understood that a clinical or biological "sample" encompasses a variety of sample types obtained from a subject and useful in an in vitro procedure, such as a diagnostic test. The definition encompasses solid tissue samples obtained during surgery, biopsy, or autopsy, and liquid samples such as blood, spinal fluid, bone marrow aspiration, fluid obtained by amniocentesis, and various subfractions, enrichments, or solubilized extracts derived from such collections. An "individual" or "subject" refers to vertebrates, particularly members of a mammalian species, such as domestic animals. sport animals, and primates, especially humans.

As used herein, the term "comprising" and its cognates are used in their inclusive sense; that is, equivalent to the term "including" and its corresponding cognates. "hEDRF" is human erythrocyte differentiation related factor, as described in this disclosure. It is understood that procedures described for making and using hEDRF and cDNA encoding it can also be applied to other peptides and polynucleotides of the invention, where appropriate.

It should be noted that, as used herein, the singular form "a", "an" and "the" include plural references unless the context clearly dictates otherwise.

### General techniques

The assembly of reagents and the carrying out of the invention will employ strategies and methods taught in this disclosure in combination with conventional techniques of molecular genetics, cell biology, biochemistry, immunology, and clinical medicine. The general techniques to be applied are within the capabilities of those skilled in the art, and are explained in standard reference publications, amongst which are the following:

Methods in molecular genetics and cell biology are described in *"Molecular Cloning: A Laboratory Manual*", 2nd Ed. (Sambrook et al., 1989); *"Oligonucleotide Synthesis*" (M.J. Gait. ed., 1984); "*Animal Cell Culture"* (R.I. Freshney, ed., 1987); the series "*Methods in Enzymology*" (Academic Press, Inc.); "*Gene Transfer Vectors for Mammalian Cells* " (J.M. Miller & M.P. Calos, eds., 1987); *"Current Protocols in Molecular Biology* " and "*Short Protocols in Molecular Biology*, *3rd Edition"* (F.M. Ausubel et al., eds., 1987 & 1995); and *"Recombinant DNA Methodology II*" (R. Wu ed., Academic Press 1995). Reagents, cloning vectors, and kits for various types of genetic manipulations referred to in this disclosure are available from commercial vendors such as BioRad, Stratagene, Invitrogen, and ClonTech.

Methods for peptide synthesis and manipulation are described in "*Solid Phase Peptide Synthesis*", (J.M. Stewart & J.D. Young, 1984); *"Solid Phase Peptide Synthesis: A Practical Approach" (E.* Atherton & R.C. Sheppard, 1989); *"The Chemical Synthesis of Peptides*" (J. Jones, International Series of Monographs on Chemistry vol. 23, 1991); and "*Solid Phase Peptide Synthesis*", (G. Barany & R.B. Merrifield, Chapter 1 of "*The Peptides",* 1979); and "*Bioconjugate Techniques"* (G.T. Hermanson, 1996).

General techniques used in raising, purifying and modifying antibodies, and the design and execution of immunoassays, the reader is referred to *Handbook of Experimental Immunology* (D.M. Weir & C.C. Blackwell, eds.); *Current Protocols in Immunology* (J.E. Coligan et al., eds., 1991); David Wild, ed., *The Immunoassay Handbook* (Stockton Press NY, 1994); and R. Masseyeff, W.H. Albert, and N.A. Staines, eds., *Methods of Immunological Analysis* (Weinheim: VCH Verlags GmbH, 1993).

### Polynucleotides of the invention and their use

This invention provides polynucleotides containing a nucleotide sequence related to the human mRNA sequence for hEDRF (SEQ. ID NO:1) and complements thereof. The human sequence is different from its mouse homologue (shown in SEQ. ID NO:3 and available as GenBank accession no. AF060220). Also provided are polynucleotides that encode hEDRF and other polypeptides of this invention within the degeneracy of the amino acid code, including complements of the polynucleotides.

Certain polynucleotides of this invention comprise at least one nucleotide sequence that is identical or partly identical to a sequence contained in SEQ. ID NO:1. The partly identical region is generally at least 10 nucleotides, and may be at least 25, 50, 100, or 400 nucleotides in length in order if increasing preference. The degree of identity in the region being compared between the sequences is typically at least 50%, and may be about 70%, 80%, 90%, 95% or 100% in order of increasing preference. Preferably, identity between two polynucleotide sequences is calculated using BLAST (Altschul, et al., 1990, *J. Mol. Biol.* **215**(3):403-410), particularly BLASTN 2 as implemented by the National Center for Biotechnology Information (NCBI), using default parameters (*e.g*., Matrix 0 BLOSUM62, +1 for match, -2 for mismatch, open and extension gap penalties of 5 and 2, respectively, gap x_dropoff 50 and word size 10). When comparison is made between polynucleotides for degree of identity, it is implicitly understood that complementary strands are easily generated, and the sense or antisense strand is selected or predicted that maximizes the degree of identity between the polynucleotides being compared.

Polynucleotides and oligonucleotides of invention (especially those below about 50 nucleotides in length) may be conveniently prepared by chemical synthesis using the sequence data provided in this disclosure. Double stranded polynucleotides of greater than about 50 nucleotides in length may also be prepared by chemical synthesis, by synthesizing oligonucleotides corresponding to segments of the desired sequence, then annealing the oligonucleotides to form a longer molecule. This method is preferred when the desired polynucleotide differs from a naturally occuring hEDRF sequence by more than a few nucleotides (*e.g.*, where the polynucleotide is designed to exploit preferred codon usage in a non-human cell, such as a bacterium). Several methods of synthesis are known in the art, including the triester method and the phosphite method. Chemical synthesis of polynucleotides generally involves iterative cycles of deprotection of the growing end of the synthetic polynucleotide, followed by chemical coupling of an additional, protected residue. In a preferred method, polynucleotides are prepared by solid-phase synthesis using mononucleoside phosphoramidite coupling units. See, for example, Beaucage et al. (Tetra. Lett. 22:1859, 1981), Kumar et al.(J. Org. Chem. 49:4905), and U.S. Patent No. 4,415,732.

Polynucleotides can also be obtained by the techniques of molecular biology. Most conveniently, polymerase chain reaction (PCR) technology is used to obtain polynucleotides of the invention. Using the sequence data provided in this disclosure, polynucleotide primers are designed that span the sequence of interest. A mRNA or cDNA template is selected from a source known or expected to express mRNA encoding hEDRF. preferably at a significant level. Suitable tissue sources include any site or source of erythrogenesis, such as human bone marrow, human fetal liver, and circulating progenitor cells. PCR techniques are well known in the art, and generally involve synthesizing cDNA if the template is mRNA, then making replicate copies of the sequence of interest from the template cDNA using one or more sets of primers, and a catalyst of polymerization, such as a reverse transcriptase or a DNA polymerase, and particularly a thermally stable polymerase enzyme. PCR methods may also be used to generate variant polynucleotides which are encompassed by the invention, as is known in the art. Methods for PCR are taught, for example, in U.S. Patent Nos. 4,683,195 and 4.683,202.

Polynucleotides of the invention may also be isolated by traditional traditional cloning methods. By this approach, polynucleotides of the invention may be isolated from cDNA or genomic libraries. As is known in the art, a cDNA (derived from a tissue known or expected to produce hEDRF, preferably at a significant level) or genomic library is probed using a labelled polynucleotide probe designed to hybridize to a polynucleotide of the invention. Clones containing DNA inserts which hybridize to the probe are selected purified, and can be cleaved to liberate polynucleotides of the invention.

Polynucleotides of this invention with encoding regions for functional protein can be used for producing such proteins by recombinant expression. This is described in the section below. Such polynucleotides also have potential for therapeutic use where the proteins they encode are also therapeutic. Accordingly, the invention also encompasses expression constructs comprising polynucleotides of the invention, as well as host cells comprising such expression constructs.

Certain polynucleotides of this invention are of interest because of their ability to hybridize to mRNA encoding hEDRF associated with cells in the differentiation pathway. This permits determining expression of hEDRF by obtaining a sample containing mRNA from a subject of interest, optionally amplifying the mRNA (or cDNA derived from the mRNA), and then determining whether a sequence encoding hEDRF is present. Preferred polynucleotides for this purpose will form a stable hybrid with any DNA or RNA present in the sample that encodes hEDRF, in preference to other DNA or RNA present in the sample.

One method of detecting hEDRF encoding sequences is using a probe which hybridizes specifically with mRNA encoding hEDRF, or cDNA derived from such mRNA. One of skill in the art will appreciate that polynucleotides with a longer matching sequence are preferred as more likely to distinguish the target sequence. Longer sequences can be incorporated with more labeling moieties per strand, and need not be as closely identical to the target in order to uniquely identify it. However, shorter sequences generally provide more tissue penetration and more rapid hybridization kinetics. Usually, a probe will comprise a label or a means by which a label can be attached, either before or subsequent to the hybridization reaction. Means for attaching labels include biotin moieties that couple with avidin or streptavidin, haptens that couple with anti-hapten antibody, and particular polynucleotide sequences (optionally on a branch or fork) that hybridize with a reagent polynucleotide having a complementary sequence, any of which ultimately lead to the attachment of a label. Suitable labels include radioisotopes, fluorochromes, chemiluminescent compounds, dyes, and proteins, including enzymes. Probes can be used for detecting specific sequences in blots of isolated mRNA or DNA. Probes can also be used to localize specific sequences in situ.

Another method of detecting hEDRF encoding sequence in a sample is to amplify the sequence in a PCR reaction, using primers specific for the encoding sequence. Preferred oligonucleotides for use as PCR primers are preferably 10 to 100 nucleotides in length and more typically 15 to 50 nucleotides in length. Nested primers can provide further levels of specificity. In comparing between samples, the amount of amplified DNA correlates with the amount of template hEDRF sequence in the original sample.

### Polypeptides of the invention

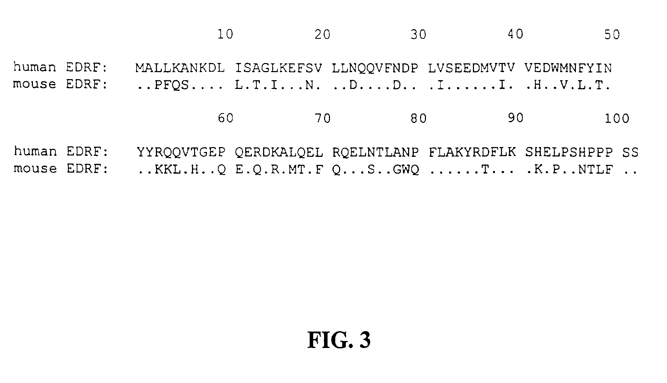

This invention provides polypeptides containing an amino acid sequence identical, partly identical, or homologous to SEQ. ID NO:2. The human sequence is different from its mouse homologue, shown in SEQ. ID NO:4.. A comparison of the two sequences is shown in FIG. 3.

Post-translational-processing of hEDRF may include cleavage of a secretion signal by signal peptidase or other proteolytic processing enzymes. A number of potential signal peptidase cleavage sites are present in the protein sequence, which may be differentially utilized according to the developmental stage and type of the cell which produces hEDRF protein. Accordingly, this invention includes polypeptides comprising residues 1-102, 6-102, 12-102, 13-102, 14-102 and 15-102 of SEQ. ID NO:2, and other fragments with desirable activity. Identification of the N-terminal of mature hEDRF from various tissues and developmental stages may be routinely determined by one of skill in the art. Generally, mature hEDRF is isolated from a solubilized, lysed or otherwise disrupted human tissue source by affinity isolation using an antibody raised against synthetic fragments from beyond residue 33. The signal peptidase cleavage site may then be determined by N-terminal amino acid sequencing and/or mass spectroscopy.

Certain polypeptides of this invention comprise at least one amino acid sequence that is identical or homologous to a sequence contained in SEQ. ID NO:2. The identical or homologous region is generally at least about 5, 8, 10, 15, 25, or 100 amino acids in length in order of increasing preference. The degree of identity in the region being compared between the sequences is typically at least 65%, 80%, 90%, 95% or 100% in order of increasing preference. Alternately, the degree of homology in the region being compared between the sequences is typically at least 85%, 90%, 95% or 100% in order of increasing preference. Particularly preferred are polypeptides with a region closely identical or homologous to a sequence contained in the mature or secreted form of hEDRF. Determination of identity and homology is preferably carried out using BLAST (Altschul, et al., 1990, *J*. *Mol. Biol.* **215**(3):403-410), particularly BLASTP 2 as implemented by the National Center for Biotechnology Information (NCBI), using default parameters (*e.g.*, Matrix 0 BLOSUM62, gap open and extension penalties of 11 and 1, respectively, gap x_dropoff 50 and wordsize 3). BLASTP 2 may be accessed via the internet at http://www.ncbi.nlm.nih.gov/BLAST/. Unless referred to as "consecutive" amino acids, a sequence optionally can contain a reasonable number of gaps or insertions that improve alignment.

Included within the invention are proteins in which one or more of the amino acids are different from that of naturally occurring hEDRF. Some amino acid substitutions are more easily tolerated. For example, substitution of an amino acid with hydrophobic side chains, aromatic side chains, polar side chains, side chains with a positive or negative charge, or side chains comprising two or fewer carbon atoms, by another amino acid with a side chain of like properties can occur without disturbing the homology of the two sequences. Methods for determining homologous regions and scoring the degree of homology are well known; see for example Altschul et al. *(infra).* Well-tolerated sequence differences are referred to as "conservative substitutions", and are preferred over non-conservative substitutions. Preferred sequences preserve the functionality of the reference polypeptide according to such criteria as affinity and specificity of receptor-ligand binding, reactivity with specific antibodies. and X-ray crystallographic structure.

The polypeptides of the invention may be prepared by any method known in the art, including, but not limited to, purification from natural sources, chemical synthesis, expression of a polynucleotide encoding the polypeptide in a cell-free translation system. or in a host cell. Short polypeptides of ≤ about 30 amino acids in length are conveniently prepared from sequence data by chemical synthesis. A preferred method is solid phase synthesis, in which the C-terminal amino acid is attached to a solid phase and the peptide is grown towards the N-terminal. The process involves reiterating the steps of deprotection of the growing protein on the solid phase and coupling the next amino acid. The added amino acids are protected other than at the 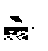 -carboxy with groups such as F-Moc, Boc, Dde, Trityl, and the like, to prevent reactions except with the solid phase peptide. After addition, the 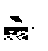 -amino group is deprotected using the appropriate reagent, and the cycle is repeated. After the peptide is complete, amino acid side chains are deprotected and the peptide is cleaved from the resin. See generally H. Dugas, C. Penney, Bioorganic Chemistry, Springer-Verlag, New York, pp 54-92 (1981).

Longer polypeptides are more conveniently prepared using a suitable recombinant expression system, in which the encoding strand a DNA sequence encoding a polypeptide of the invention is operatively linked to a suitable promoter, inserted into an expression vector, and transfected into a suitable host cell. The host cell is then cultured under conditions that allow transcription and translation of the protein, which is subsequently recovered and purified. See Maniatis, et al., Molecular Cloning: A Laboratory Manual, Cold Spring Harbor Press, Cold Spring Harbor Laboratory, Cold Spring Harbor, N.Y. (1989), or Current Protocols in Molecular Biology (1989) and supplements.

Proteins of this invention may be made either by direct expression, or as part of a larger fusion protein, followed by enzymatic or chemical cleavage of the desired portion. The proteins of the invention may be full length (*i.e.*, 1-102 of SEQ. ID. NO. 2), a processed form (6-102, 12-102, 13-102, 14-102 and 15-102 of SEQ. ID NO:2) or a fragment thereof. A variety of peptidases (e.g. trypsin) which cleave a polypeptide at specific sites or digest the peptides from the amino or carboxy termini (e.g. diaminopeptidase) of the peptide chain are known. Furthermore, particular chemicals (e.g. cyanogen bromide) will cleave a polypeptide chain at specific sites. See e.g., Carter P., Site Specific Proteolysis of Fusion Proteins, Chapter 13 in Protein Purification: From Molecular Mechanisms to Large Scale Processes, American Chemical Soc., Washington, D.C. (1990).

Generally, a polynucleotide encoding a protein of the invention (or a protein of the invention fused to other sequence) is inserted into an appropriate recombinant DNA expression vector using appropriate restriction endonucleases. The restriction endonuclease sites may be naturally occuring or synthetic sites that have been introduced by any method known in the art, such as site-directed mutagenesis, PCR or ligation of linker/adapters to the polynucleotide. Alternately, the polynucleotide may be a synthetic sequence, designed to incorporate convenient restriction enzyme sites and/or optimize codon usage for the intended host cell. The particular endonucleases employed will be dictated by the restriction endonuclease cleavage pattern of the parent expression vector to be employed. The choice of restriction sites is made so as to properly orient the coding sequence with control sequences to achieve proper in-frame reading and expression of the protein.

The polynucleotide may be inserted into any appropriate expression vector. Expression vectors may be found in a number of forms, including, but not limited to, plasmid, cosmid, yeast artificial chromosome (YAC), and viral. In general, the expression vector will contain an autonomous replication site that is active at least in the organism in which the vector is propagated, and frequently also in the recombinant host cell. The expression vector will also typically include marker sequences which are capable of providing phenotypic selection in transformed cells, such as positive selection markers, such as an antibiotic resistance genes (*e.g.*, *bla*, tet^{R}, or hyg^{R}) or genes which complement an auxotrophy (*e.g.*, trp or DHFR) and/or negative selection markers such as herpes simplex virus 1 thymidine kinase. The expression vector will also include necessary sequences for initiation and termination of transcription (*e.g.*, promoter, Shine-Dalgarno sequence, ribosome binding site, transcription termination site) and may optionally contain sequences which modulate transcription *(e.g.,* SV40 enhancer or *lac* repressor), and may also contain sequences which direct processing, such as an intron or a polyadenylation site, as necessary.

The polynucleotide of the invention is inserted into the expression vector in the proper orientation and relationship with the expression vector's transcriptional control sequences to allow transcription from the promoter and ribosome binding site, both of which should be functional in the host cell in which the protein is to be expressed. The transcriptional control sequences are preferably inducible (*i.e.*, can be modulated by altering the culture conditions, such as the *lac* operon for E. *coli* or the metallothionein promoter for mammallian cells). An example of such an expression vector is a plasmid described in Belagaje et al., U.S. Pat. No. 5,304,493. The gene encoding A-C-B proinsulin described in the reference can be removed from the plasmid pRB182 with restriction enzymes NdeI and BamHI. The genes encoding the protein of the present invention can be inserted into the plasmid backbone on a NdeI/BamHI restriction fragment cassette.

Microbial hosts are normally preferred for recombinant expression of the proteins of the invention. and any commonly used microbial host, including E. *coli* such as W3110 (prototrophic, ATCC NO. 27325), Bacillus subtilis, and other enterobacteriaceae such as Salmonella typhimurium or Serratia marcescans, and various pseudomonas species may be used. Alternately, eukaryotic host cells, including yeast such as *Saccharomyces cerevisiae*, *Schizosaccharomyces pombe*, as well as higher eukaryotes such as fungal cells, plant cells, insect cells (*e.g.*, Sf9), and mammalian cells *(e.g.,* COS, CHO) may be used.

The completed expression construct is introduced into the recombinant host cell by any appropriate method known in the art, such as CaCl₂ transfection, Ca₂PO₄ transfection, viral transduction, lipid-mediated transfection, electroporation, ballistic transfection, and the like. After introduction of the expression construct, the recombinant host cell is generally cultured under appropriate conditions to select for the presence of the expression construct *(e.g.,* cultured in the presence of ampicillin for a bacterial host with an expression construct containing *bla*), or alternately may be selected for expression of the protein by any appropriate means (*e.g.*, fluorescence activated cell sorting, FACS, using a hEDRF-specific antibody).

After selection and appropriate isolation procedures (*e.g.*, restreaking or limiting dilution cloning), the recombinant host cells are cultured at production scale, using any appropriate technology known in the art. If the promoter/enhancer in the expression vector is inducible, the expression of the protein is induced after the culture reaches an appropriate cell density, otherwise the cells are grown until they reach appropriate density for harvet. Harvesting of the recombinant proteins of the invention will depend on the exact nature of the recombinant host cells, the expression construct, and the polynucleotide encoding the protein of the invention, as will be apparent to one of skill in the art. For expression constructs that result in a secreted protein, the protein is normally recovered by removing media from the culture vessel, while expression constructs that result in intracellular accumulation of the protein generally require recovery and lysis of the cells to free the expressed protein.

Proteins which are expressed in high-level bacterial expression systems characteristically aggregate in granules or inclusion bodies which contain high levels of the overexpressed protein. The protein aggregates are solubilized to provide further purification and isolation of the desired protein product, for example, using strongly denaturing solutions such as guanidinium-HCl, possibly in combination with a reducing agent such as dithiothreitol (DTT). The solubilzed protein is recovered in its active form after a "refolding" reaction, in which generally involves reducing the the concentration of the denaturant and adding oxidizing agent. Protocols which are considered generally applicable for the refolding of proteins are well known in the art, and are disclosed in, for example, U.S. Patents Nos. 4,511,502, 4,511,503, and 4,512,922.

Proteins of the invention may be utilized for the production of antibodies, as described below.

Proteins of the invention are also useful in screening asays to identify pharmaceutical compounds of interest. Proteins of the invention are preferably used as a "primary" screen to identify compounds for further characterization, by screening for compounds which bind to hEDRF.

### Antibodies

The invention provides antibodies specific for hEDRF and the other polypeptides disclosed herein.

Polyclonal antibodies of this invention are typically raised by administration of hEDRF or one of the polypeptides of this invention in an immunogenic form to a mammalian host. It is preferable to administer the polypeptide with an adjuvant such as a water-in-oil immersion, particularly Freund's complete adjuvant for the first administration, and Freund's incomplete adjuvant for booster doses. The preparation is typically administered in a variety of sites, and typically in two or more doses over a course of at least 4 weeks. Serum is harvested and tested for the presence of specific antibody.

Monoclonal antibodies of this invention can be prepared by a number of different techniques. For hybridoma technology, the reader is referred generally to Harrow & Lane (1988), U.S. Patent Nos. 4,491,632, 4,472,500, and 4,444,887, and *Methods in Enzymology*, 73B:3 (1981). Traditional monoclonal antibody technology involves the immortalization and cloning of an antibody-producing cell recovered from an animal that has been immunized as described in the preceding paragraph. The cell may be immortalized by, for example, fusion with a non-producing myeloma, infection with Epstein Barr Virus, or transformation with oncogenic DNA. The treated cells are cloned and cultured, and clones are selected that produce antibody of the desired specificity. Specificity testing is performed on culture supernatants by a number of techniques, such as using the immunizing antigen as the detecting reagent in an immunoassay. A supply of monoclonal antibody from the selected clone can then be purified from a large volume of culture supernatant, or from the ascites fluid of suitably prepared host animals injected with the clone.

Alternative methods for obtaining monoclonal antibodies involve contacting an immunocompetent cell or viral particle with a protein of the invention. In this context, "immunocompetent" means that the cell or particle has expressed or is capable of expressing an antibody specific for the antigen without further genetic rearrangement, and can be selected from a cell mixture by presentation of the antigen. Immunocompetent eucaryotic cells can be harvested from an immunized mammalian donor, or they can be harvested from an unimmunized donor and prestimulated in vitro by culturing in the presence of immunogen and immunostimulatory growth factors. Cells of the desired specificity can be selected by contacting with the immunogen under culture conditions that result in proliferation of specific clones but not non-specific clones. Immunocompetent phage may be constructed to express immunoglobulin variable region segments on their surface. See Marks et al., *New Engl. J Med.* **335**:730, 1996; International Patent Applications Nos. 94/13804, 92/01047, 90/02809; and McGuinness et al., *Nature Biotechnol*. **14**:1149, 1996. Phage of the desired specificity may be selected, for example, by adherence to hEDRF (or an immunological equivalent) attached to a solid phase, and then amplified in *E. coli.*

Antibody can be purified from serum, cell supernatants, lysates, or ascites fluid by a combination of traditional biochemical separation techniques, such as ammonium sulfate precipitation, ion exchange chromatography on a weak anion exchange resin such as DEAE, hydroxyapatite chromatography, and gel filtration chromatography.

Antibodies obtained are preferably screened or purified not only for their ability to react with hEDRF, but also for a low cross-reactivity with potential cross-reacting substances also present in samples of diagnostic interest. Unwanted activity can be adsorbed out of polyclonal antisera, if necessary, using the cross-reacting substance or an antigen extract from a population of cells depleted for the protein of interest.

The epitope to which a particular antibody binds can be mapped by preparing fragments and testing the ability of the antibody to bind. For example, sequential peptides of 12 amino acids are prepared covering the entire sequence, and overlapping by 8 residues. The peptides can be prepared on a nylon membrane support by F-Moc chemistry, using a SPOTS™ kit from Genosys according to manufacturer's directions. Prepared membranes are then overlaid with the antibody, washed, and overlaid with 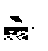 -galactose conjugated anti-human IgG. The test is developed by adding the substrate X-gal. Positive staining indicates an antigen fragment recognized by the antibody. The fragment can then be used to obtain other antibodies recognizing the epitope of interest. Two antibodies recognizing the same epitope will compete for binding in a standard immunoassay.

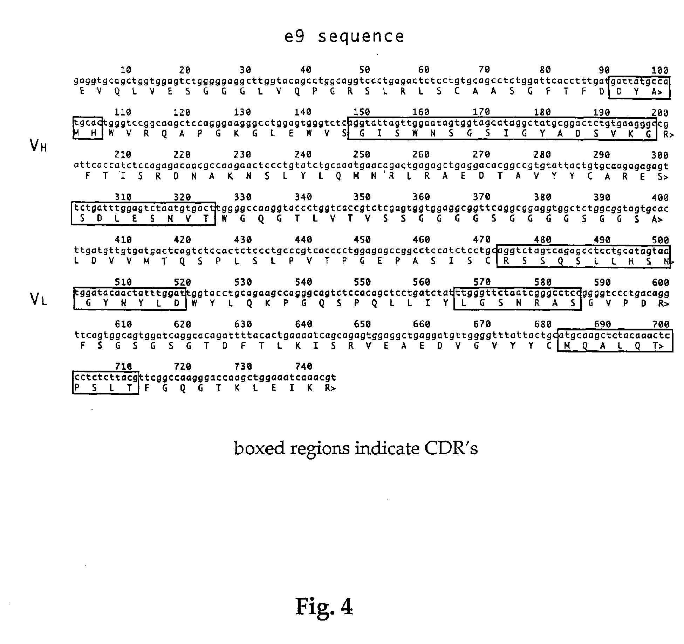

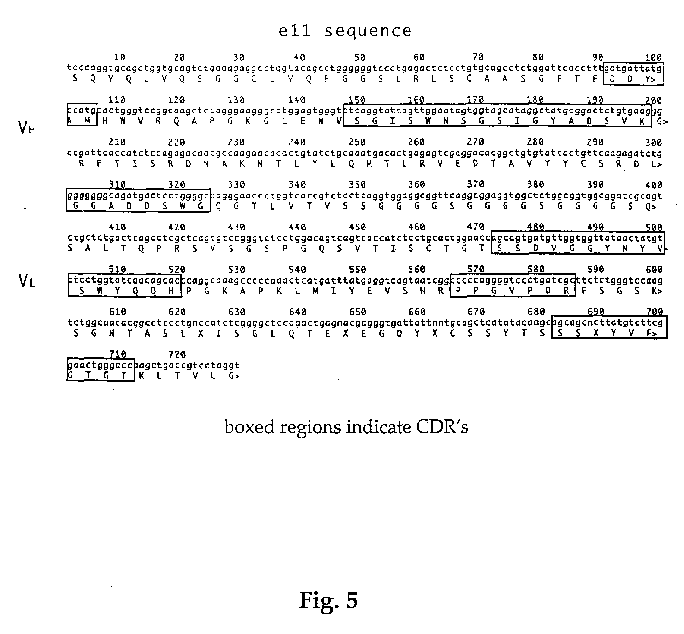

Certain preferred antibody embodiments of the invention include antibodies which comprise the V_{H} and/or V_{L} of antibodies e9 or e11. Additional preferred antibodies include antibodies which comprise one or more complementarity determining regions (CDRs) from antibodies e9 or e11. Antibodies e9 and el were isolated from a human scFv library, as described in Example 2. The sequences of e9 and e11 are shown in FIG. 4 and FIG. 5, and the CDRs are boxed in each figure.

The antibodies can be used for determining the presence of the proteins of the invention in samples from human subjects by, for example, using the antibodies in any immunoassay known in the art. Such methods typically comprise obtaining a sample from the subject, contacting protein in the sample with the antibody under conditions permitting the formation of antibody-antigen complexes, and determining complexes formed. This can be done by using the antibody in a fluid phase immunoassay, or in immunohistochemical staining. Immunoassays may be separation type assays, in which complexed antibody is separated from excess reagent. One example is a sandwich or precipitation assay, in which the antigen is captured or precipitated using one antibody, and detected using a second antibody equipped with a radiolabel such as ¹²⁵I, or an enzyme label such as alkaline phosphatase. Alternatively, antigen-antibody complexes formed in the reagent mixture can be separated by physicochemical properties; for example, by gel filtration. Immunoassays may also be of the homogeneous type, in which the binding of antigen to the first antibody provides a measurable signal, such as a color change. A particularly powerful homogeneous assay system is the cloned enzyme donor immunoassay (CEDIA®), described in U.S. Patent No. 4,708,929. Immunohistochemistry involves overlaying sections of tissue sample with the specific antibody, washing, and then detecting any antibody which has bound to antigen in the tissue. This is done, for example, by overlaying with an anti-immunoglobulin reagent having a suitable label. The location of the antigen in the tissue can then be determined by label detection at a microscopic level.

Detection or quantitation of hEDRF in a human subject can be performed whenever it is desirable to identify or quantitate erythroid cells

Quantitation of hEDRF at the immunohistochemical level may be of value in evaluating the potential of the tissue site to contribute towards the production of new erythroid cells.

Antibodies of this invention can also be used for purifying hEDRF and other peptides from human tissue or recombinant sources by solid phase or fluid phase immunoaffinity techniques. In a typical procedure, the antibody is attached to a solid phase, such as CNBr-activated Sepharose®. The source of the peptide is contacted with the solid phase antibody, and the solid phase is washed to remove contaminants. The peptide is then recovered using a suitable eluting solvent, such as 1 M KSCN or 0.1 M glycine buffer. pH 2.5.

### EXAMPLES

### Example 1: Expression Pattern of hEDRF

The expression pattern of hEDRF was investigated by various hybridization techniques. The entire clone, designated 345-7a, was used for hybridization studies.

Northern blotting was used to determine the tissue distribution of hEDRF expression. RNA was isolated from a number of sources (in some cases, pools of sources), separated on agarose gels, transferred to nitrocellulose. Blots were and probed with random-primed. ³²P-labeled 345-7a using standard hybridization and wash conditions.

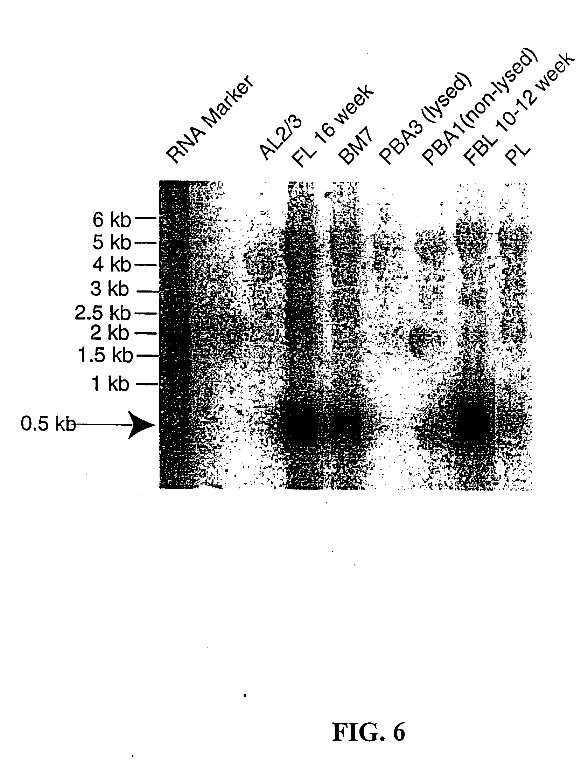

FIG. 6 shows a representative Northern blot. From left to right, the lanes were loaded with an RNA molecular weight marker, a blank, a pool of adult liver RNA (AL 2/3), a pool of week 16 fetal liver RNA, a pool of adult bone marrow RNA (BM7), a pool of adult peripheral blood RNA (PBA3) prepared from a lysed sample, a pool of adult peripheral blood RNA (PBA1) prepared from a non-lysed sample, a pool of fetal blood RNA from 10 to 12 week fetal liver, and a pool of RNA from term placenta. Note the strong signal in the fetal liver and fetal blood from liver lanes, as well as the signal from adult bone marrow, indicating an expression pattern associated with erythroid cells.

### Example 2: Antibodies to hEDRF

Purified 345-7a peptide (NH₂-Asn-Tyr-Tyr-Arg-Gln-Gln-Val-Thr-Gly-Glu-Pro-Gln-Glu-Are-Asp-Lys-Ala-Leu-Gln-Glu-Leu-Arg-Gln-Glu-Leu-Asn-Thr-Leu-Ala-Asn-Pro-Phe-Leu-Ala-Lys-Tyr-Arg-Asp-Phe-Leu-Lys-Ser-His-Glu-Leu-Pro-Ser-His-COOH, SEQ ID NO:9) was used to select antibodies specific for hEDRF from a naïve human scFv library. The library was Griffin.1 as described in Griffiths et al. (1993, *EMBO J*. **12**(2):725-734).

345-7a peptide was biotinylated to a level of three biotin molecules per peptide using the EZ-Link NHS-ester biotinylation kit from Pierce (#21420) according to the manufacturer's instructions. The biotinylated peptide was bound to streptavidin-coated magnetic particles (M-280, Dynal) for use as an antibody selection reagent. 200 µl of magnetic particles were incubated with 100 µg of biotinylated 345-7a peptide in 1 ml of phosphate buffered saline (PBS) with 1% bovine serum albumin (BSA) for one hour at room temperature. Unbound peptide was removed by washing the peptide/streptavidin-coated magnetic particles three times with PBS containing 0.05% Tween 20. The antibody selection reagent was resuspended in 100 µl of PBS.

500 µl of the antibody library (5 x 10¹² pfu) were incubated with 200 µl of unconjugated streptavidin magnetic particles to adsorb out viral particles which bind the particles. The particles were removed, and the first round of selection was performed by incubating adsorbed phage with the antibody selection reagent for two hours with rocking. The antibody selection agent (with attached phage) was separated from the solution by magnetic affinity and washed 20 times (in PBS with 0.05% Tween 20). The adherent phage were eluted from the antibody selection reagent by incubation in 100 µl of 100 mM triethylamine for five minutes, separated from the magnetic particles, and neutralized by the addition of 20 µl 1 M Tris, pH 7.4 . The phage solution was used to infect *E. coli* TG1 cells, which were plated on media containing ampicillin. Colonies were enumerated, then the pool of selected phage was amplified by scraping the plates, incubating the resulting culture in 2 x YT media plus ampicillin and glucose for one hour followed by a further hour of incubation in the presence of hlper phage M13K07, then diluting the culture in 10 volumes of 2 x YT with ampicillin and kanamycin (50 µg/ml) and incubating for 16-18 hours at 30° C. Phage in the culture supernatant were precipubated by the addition of 1/5 volume of 20% polyethyleneglycol (PEG), 2.5 M NaCl on ice for 30 minutes. Precipitated phage was collected by centrifugation (10,000 x g for 20 minutes), then resuspended in PBS to form a concentrated phage stock.

A second round of selection was performed on the amplified phage, using avidin magnetic particles to minimize selection of phage which bind to streptavidin. After amplification, the phage were run through a third round of selection, returning to streptavidin magnetic particles.

Individual colonies from the third round of selection were tested in an ELISA assay using the biotinylated 345-7a peptide bound to streptavidin-coated plates. Streptavidin-coated 96 well plates were obtained from Pierce, and incubated with 1 µg/ml biotinylated 374-7a peptide. Individual phage clones were grwon in 96 will plates by incubating single colonies in 2 xYT plus ampicillin and glucose for two hours, adding helper phage, then growing the infected cells overnight at 30° C in 2 x YT plus ampicillin and kanamycin. 150 µl of phage supernatant was incubated for 2 hours in each well (plates which had not been coated with 345-7a peptide served as controls). After incubation with phage supernatants, the wells were washed PBS, and detected by incubation with horseradish peroxidase (HRP) conjugated anti-M13 antibody (Sigma) one hour, followed by development with the chromogenic substrate 2,2'-Azino-bis(3-ethylbenzthiazoline-6-sulfonic acid) (ABTS, Sigma).

Positive clones were first analyzed by a PCR/restriction fingerprint analysis. PCR primers directed to vector backbone sequences were used to amplify DNA from each of the phage which tested positive in the ELISA, and the amplified DNA was digested with restriction enzyme Bst N1. Two distinct clones were identified by this procedure, and designated e9 and e11. Complete DNA sequencing of the variable regions of the scFv genes carried by representative phage from each of the two different clones the distinctness of the two clones.

e9 and e11 were tested for binding of the 345-7a peptide in solution. e9 and e11 scFv were adsorbed to nickel-charged NTA agarose beads via the His₆ tag on the scFv provided by the vector. The beads were washed, then incubated in a solution containing 10 µg/ml 345-7a peptide, then washed again. Bound 345-7a peptide was detected with rhodamine-labeled streptavidin.. Both e9 and el 1 adsorbed beads bound 345-7a peptide.

e11 was converted from an scFv to a recombinant Fab by sequentially cloning its VH, then its VL genes into the Fab expression vector VODOX-1. Fab e11 retained 345-7a binding activity as measured by the solution binding assay described above.

Fab e11 was tested for binding to cells and tissues. Fetal liver cells from 18 week human fetal livers, unfixed or fixed/permeabilized with the Fix & Perm kit (#GAS003) from Caltag Laboratories in accordance with the manufacturers instructions, were incubated with e11 Fab at 10 µg/ml or with anti-transferrin ( 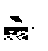 - Tfr) Fab. Binding was detected by rhodamine-labeled secondary antibodies. e11 labeled only fixed/permeabilized cells indicating that it is specific for a cytoplasmic protein. The 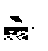 -Tfr Fab labeled both unfixed and fixed/permeabilized cells, with a punctate staining pattern indicative of a cell surface protein.

All publications and patent applications mentioned in this specification are herein incorporated by reference to the same extent as if each individual publication or patent application were explicitly and individually incorporated by reference.

The invention now being fully described, it will be understood that description provided in the specification is intended to illustrate but not limit the invention. Those skilled in the art will readily appreciate that modifications can be incorporated without departing from the spirit the appended claims.

## Claims

1. A polypeptide comprising at least 5 consecutive amino acids that are identical to consecutive amino acids shown in SEQ. ID NO:2, but not in SEQ. ID NO:4.

2. A polypeptide comprising at least 8 consecutive amino acids that are identical to consecutive amino acids shown in SEQ. ID NO:2.

3. The polypeptide of claim 2, comprising an amino acid sequence to which is at least about 65% identical to amino acids 15 to 102 of SEQ. ID NO:2.

4. A polypeptide which is at least about 65% identical to the sequence shown in SEQ. ID. NO. 2.

5. A polypeptide which is at least about 85% homologous to the sequence shown in SEQ. ID. NO. 2.

6. An isolated polynucleotide encoding a polypeptide which is 65% identical to amino acids 15 to 102 of SEQ. ID NO:2 or the complement of said polynucleotide.

7. An isolated polynucleotide comprising at least 25 consecutive nucleotides that are identical to consecutive amino acids shown in SEQ. ID NO. 1 or the complement of said isolated polynucleotide.

8. The isolated polynucleotide of claim 7, to which at least about 100 consecutive nucleotides of SEQ. ID NO:1 is at least about 90% identical or the complement of said isolated polynucleotide.

9. A host cell comprising a polynucleotide encoding a polypeptide which is 65% identical to amino acids 15 to 102 of SEQ. ID NO:2.

10. A method for producing a polypeptide which is at least about 65% identical to the sequence shown in SEQ. ID. NO. 2.polypeptide, comprising expressing a polynucleotide which encodes said polypeptide in a host cell.

11. A method for detecting expression of human erythrocyte differentiation related factor (hEDRF) in a human subject, comprising the steps of:
a) obtaining a sample from the subject containing DNA or RNA, and optionally amplifying the DNA or RNA;
b) contacting the DNA or RNA with a polynucleotide according claim 11;
c) detecting said polynucleotide.

12. An antibody specific for a polypeptide which is at least about 65% identical to the sequence shown in SEQ. ID. NO. 2.

13. A method for detecting human erythrocyte differentiation related factor (hEDRF) in a human subject, comprising the steps of:
a) obtaining a sample from the subject containing protein;
b) contacting the protein with an antibody specific for a polypeptide which is at least about 65% identical to the sequence shown in SEQ. ID. NO. 2; and
c) detecting bound antibody.
